# EUROPÄISCHE PATENTSCHRIFT

(11) **EP 0 625 358 B1**
(45) Veröffentlichungstag und Bekanntmachung des Hinweises auf die Patenterteilung: **10.07.1996**
(21) Anmeldenummer: 93810371.0
(22) Anmeldetag: 19.05.1993
(51) Int. Cl.: A61M 25/01

(54) **Führungsdraht**
Guide wire
Fil de guidage

(43) Veröffentlichungstag der Anmeldung: 23.11.1994
(73) Patentinhaber: SCHNEIDER (EUROPE) AG, CH-8180 Bülach (CH)
(72) Erfinder: Sauter, Herbert, CH-8185 Winkel-Rüti (CH)
(74) Vertreter: Groner, Manfred

(56) Entgegenhaltungen:
- EP-A- 0 318 046
- EP-A- 0 419 277
- WO-A-92/19151
- US-A- 4 748 986
- US-A- 4 922 924
- Spring Design and Application, edited by Nicholas P. Chironis,McGRAW-HILL Book Company,Inc., New York, Toronto, London, 1961;Seite 307

## Beschreibung

Die Erfindung betrifft einen Führungsdraht nach dem Oberbegriff des unabhängigen Patentanspruchs 1.

Ein Führungsdraht dieser Art ist aus der EP-A-0 419 277 bekannt geworden. Zum Verbinden zweier Teile der Spirale miteinander ist als Verbindungsmittel ein Stück eines Schlauches vorgesehen, in dessen Aussenseite von den beiden Enden her je eine spiralförmige Nut eingearbeitet ist. In jede Nut ist ein Ende der zu verbindenden Teile eingedreht. Bei hohen Anforderungen an eine gleichbleibende Flexibilität des Führungsdrahtes und im Hinblick auf die kleinen Abmessungen des Verbindungsmittels dürfte dieses schwierig herzustellen und zu montieren sein. Dies umsomehr, als auch die Nuten für die Aufnahme der Spiralenden sehr präzise gearbeitet sein müssten. Das Anbringen einer Lötstelle zur Sicherung der Verbindung bei einer Torsionsbeanspruchung wäre nicht ohne weiteres möglich.

Zum Einführen eines Ballonkatheters in ein Blutgefäss, wird der Führungsdraht mit Hilfe eines Führungskatheters in das Blutgefäss gelegt und der Ballonkatheter auf den Führungsdraht aufgeschoben und im Gefäss soweit vorgeschoben, bis die zur Behandlung vorgesehene Stellung erreicht ist. Beim Legen des Führungsdrahtes ist es wichtig, dass sein distales Ende im Blutgefäss steuerbar und auf dem Röntgenbildschirm sichtbar ist. Hierbei soll es zudem möglich sein, mit Hilfe eines Kontrastmittels die zu behandelnde Stelle des Blutgefässes sichtbar zu machen. Damit das distale Ende des Führungsdrahtes die Sichtbarkeit des Gefässes möglichst wenig behindert, ist bei den bekannten Führungsdrähten dieser Art lediglich ein vorderer Bereich der Spirale für Röntgenstrahlen undurchlässig und somit gut sichtbar ausgebildet. Die beiden Teile der Spirale müssen sicher miteinander verbunden sein, so dass ein Bruch der Spirale während einer Behandlung ausgeschlossen werden kann. Andererseits soll die Verbindung dieser beiden Teile die Steuerbarkeit und die Flexibilität des Führungsdrahtes nicht beeinträchtigen.

Beim Führungsdraht nach der US-A-4,922,924 sind die beiden Teile der Spirale über einige gedehnte Windungen ineinandergedreht. An den Enden des bifilaren Bereiches sind die beiden Teile miteinander und mit dem zentralen Schaft verlötet. In die distale Lötstelle ist ein Band eingesetzt, das am anderen Ende mit der Spitze des Führungskatheters verbunden ist. Dieses Band soll sicherstellen, dass die Spirale in eine bestimmte Form gebogen werden kann und dass im Fall eines Bruches der Spirale der distale Teil der Spirale sich vom Schaft nicht loslösen kann.

Einen ähnlichen Führungsdraht zeigt die US-A-4,748,986, bei dem ebenfalls ein distaler Teil der Spirale in einen proximalen Teil eingedreht und mit diesem sowie mit dem Schaft verlötet ist. Die Lötstelle erstreckt sich hier über den gesamten bifilaren Bereich und ebenfalls ist bei diesem Führungsdraht ein Band vorgesehen, das die Spitze mit dem Schaft verbindet.

Die kritischen Stellen bei diesen Führungsdrähten befinden sich jeweils am proximalen und am distalen Ende des bifilaren Bereiches. Dort liegen die Windungen der Spirale um den Drahtdurchmesser der Spirale auseinander, damit die jeweils andere Spirale eingedreht werden kann. Dieser Spalt ist im bifilaren Bereich durch die jeweils zweite Spirale ausgefüllt. In einer gewissen Entfernung vom bifilaren Bereich ist der Spalt wieder normalisiert und auf einem kleineren Mass. Im Uebergangsbereich jedoch zwischen bifilarem Abschnitt und der Spirale mit normalem Spaltmass entsteht eine knickempfindliche Stelle, weil dort die zweite Spirale fehlt, der Spaltabstand aber noch nicht normalisiert ist.

Ein anderes Problem entsteht bei der Montage der Spiralen. Sie müssen beim Ineinanderdrehen sehr exakt ausgerichtet werden und so gehalten werden, damit beide Spiralen im montierten Zustand genau fluchtend liegen und zusammen nach dem Löten einen glatten kontinuierlichen Führungsdraht ohne Absätze ergeben.

Durch die WO 92/04072 ist ein Führungsdraht bekannt geworden, der am distalen Ende zwei koaxial zueinander angeordnete Spiralen aufweist. Von diesen ist eine kleinere innere Spirale an einem Ende mit dem Schaft verlötet. Die andere grössere Spirale ist am distalen Ende mit einer Spitze des Führungsdrahtes und am proximalen Ende mit dem Schaft sowie einem proximalen Teil der Spirale verlötet. Führungsdrähte mit zwei koaxialen Spiralen sind zudem durch die US-A-5,144,959 und durch US-A-5,063,935 bekannt geworden. Die innere Spirale ist jeweils am proximalen Ende mit dem Schaft und am distalen Ende mit der Spitze des Führungsdrahtes verbunden. Schliesslich zeigt die WO-A-92/19 151 einen Führungsdraht, bei dem eine polymere Umhüllung und eine Spirale durch eine in diese endseitig eingestechte Spirale verbunden sind.

Der Erfindung liegt die Aufgabe zugrunde, einen Führungsdraht der genannten Art zu schaffen, der eine gleichbleibende Flexibilität auch im Bereich der Verbindungsstelle aufweist, und der einfach herzustellen und trotzdem sicher und gut in Blutgefäße einschiebbar ist.

Die Aufgabe ist beim gattungsgemässen Führungsdraht gemäss Kennzeichen des Anspruchs 1 gelöst. Die aus drei Teilen zusammengesetzte Spirale verhält sich bezüglich ihrer Flexibilität im wesentlichen wie eine aus einem einzigen Draht gewickelte Spirale. Der vergleichsweise kurze röntgensichtbare Bereich ist scharf abgegrenzt. Die beiden gegenüberliegenden Enden der beiden Teile der Spirale können beim erfindungsgemässen Katheter stumpf aneinanderliegen. Die mechanischen Eigenschaften und insbesondere die Flexibilität der Spirale sind beim erfindungsgemässen Führungsdraht auch im Bereich der Verbindungsstelle der beiden Teile über die gesamte Länge der Spirale weitgehend gleich, so dass somit die Verbindungsstelle die Steuerbarkeit des Führungsdrahtes nicht beeinträchtigt. Ebenfalls ist die Aussenseite der Spirale im wesentlichen über ihre gesamte Länge weitgehend gleich und bei der Montage werden die Spiralen selbstätig zentriert. Weitere vorteilhafte Merkmale der Erfindung ergeben sich aus den abhängigen Ansprüchen, der nachfolgenden Beschreibung sowie der Zeichnung.

Ein Ausführungsbeispiel der Erfindung wird nachfolgend anhand der Zeichnungen näher erläutert. Es zeigen:
- Fig. 1a: im stark vergrössertem Massstab eine Ansicht eines teilweise längsgeschnittenen erfindungsgemässen Führungsdrahtes,
- Fig. 1b: eine Teilansicht des Führungsdrahtes gemäss Fig. 1a, und
- Fig. 2a bis 2i: schematische Darstellungen des distalen Endes des Führungsdrahtes zur Erläuterung des Verfahrens zu einer Herstellung.

Der Führungsdraht weist eine Spirale 6 auf, die auf das distale Ende eines Schaftes 1 aufgeschoben ist und die an diesem Schaft 1 am distalen Ende durch eine vorne abgerundete Spitze 5 und am proximalen Ende durch eine Lötstelle 16 mit dem Schaft 1 verbunden ist. Die Gesamtlänge des Führungsdrahtes bzw. des Schaftes 1 beträgt beispielsweise 175 cm, während die Länge der Spirale 6 beispielsweise etwa 30 cm beträgt.

Der drahtförmige, teflonbeschichtete (Teflon ist ein eingetragenes Warenzeichen) Schaft 1 weist proximal eines sich konisch verjüngenden Bereichs 2 einen kreisförmigen Querschnitt mit einem Durchmesser von etwa 0,15 mm auf. Zwischen dem konischen Bereich 2 und einem flachgepressten distalen Bereich 4 weist der Schaft 1 in einem Bereich 3 ebenfalls einen einheitlichen kreisförmigen Querschnitt auf, dessen Durchmesser beispielsweise 0,075 mm beträgt. Im Bereich 4 ist der Schaft beispielsweise auf einer Länge von etwa 2 cm auf eine Dicke von beispielsweise 0,05 mm flachgepresst. Durch das Flachpressen des Schaftes 1 an seinem distalen Ende wird die Torsionsübertragung erhöht und gleichzeitig die Ermüdungsgefahr verringert.

Der Führungsdraht ist aussenseitig mit Ausnahme eines Bereiches 17 in an sich bekannter Weise mit einer Teflonbeschichtung 7 versehen. Diese Beschichtung 7 vermindert die Reibung des Führungsdrahtes, wenn dieser in einem hier nicht gezeigten an sich bekannten Führungskatheter verschoben wird.

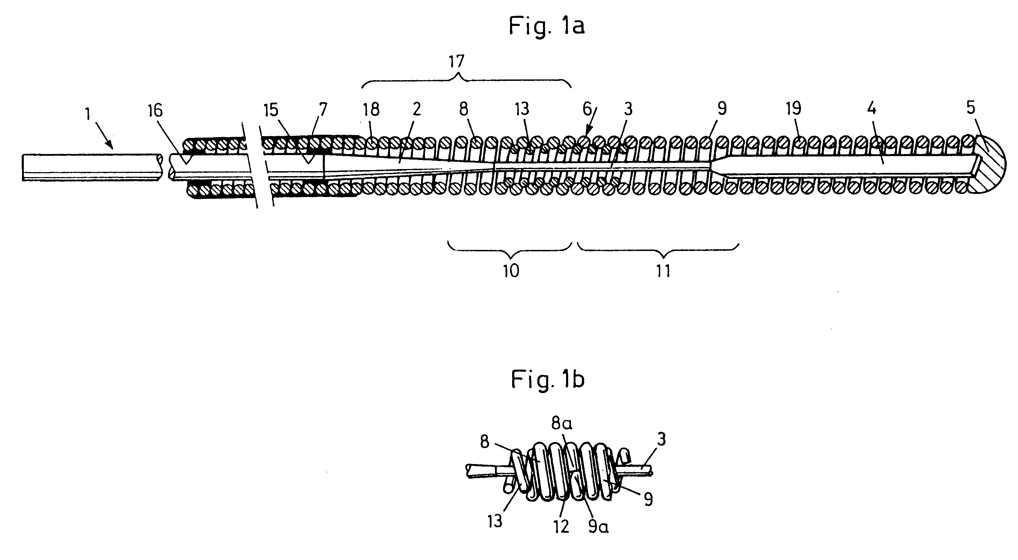

Die Spirale 6 besteht aus einem proximalen Teil 8 und einem distalen Teil 9, die mittels einer Verbindungsspirale 13 unlösbar miteinander verbunden sind. Der proximale Teil 8 ist aus einem rostfreien flexiblen Stahldraht 18 mit einem Durchmesser von ca. 0,075 mm gewickelt und anschliessend mit Teflon oder einem anderen gut gleitenden Kunststoff beschichtet. In einem Bereich 10 von beispielsweise acht bis zehn Windungen ist der Teil 8 wie aus Fig. 1a ersichtlich etwas gedehnt, die genannten Windungen sind hier somit nur leicht geöffnet. Der distale Teil 9 ist aus einem vergoldeten ca. 0,075 mm starken Wolframdraht in einem Aussendurchmesser von ca. 0,35 mm gewickelt. Dieser Teil 9 ist aussenseitig vorzugsweise nicht mit Teflon beschichtet. Der Teil 9 ist in seiner ganzen Länge, über alle Windungen, leicht geöffnet, um die Flexibilität des Teil 9 zu erhöhen.

Die Verbindungsspirale 13 ist mit einem Ende innenseitig in den Teil 8 und mit dem anderen Ende ebenfalls innenseitig in den Teil 9 eingedreht. Die Verbindungsspirale 13 weist beispielsweise acht Windungen auf, die ähnlich einem Gewinde in die Teile 8 und 9 eingreifen. Die Verbindungsspirale 13 ist aus einem vergoldeten Wolframdraht mit einem Aussendurchmesser von ca. 0,05 mm auf einem Wickeldorn mit einem Aussendurchmesser von ca. 0,1 mm hergestellt. Sie ist ebenfalls in Längsrichtung etwas gedehnt. Vorzugsweise über ihre gesamte Länge ist die Verbindungsspirale 13 durch Silberlot mit den Teilen 8 und 9 fest verbunden. Der Teil 9 und die Verbindungsspirale 13 sind röntgenographisch gut sichtbar, besonders auch durch die Materialanhäufung durch die Spirale 13, während der proximale Teil 8 im Gegensatz dazu röntgenographisch nicht sichtbar ist. Beim Einführen der distalen Spitze des Führungsdrahtes kann somit der Teil 9 röntgenographisch nicht beobachtet werden. Wesentlich ist, dass die Flexibilität der Spirale 6 durch die Verbindungsspirale 13 und ihre Verlötung mit den Teilen 8 und 9 nicht wesentlich beeinträchtigt wird. Die Spirale 6 hat somit bezüglich ihrer Flexibilität ähnliche Eigenschaften wie eine nicht zusammengesetzte Spirale. Wie die Fig. 1b zeigt, sind die Enden 8a und 9a der proximalen und distalen Teile 8 und 9 stumpf aneinandergelegt, so dass die beiden Drähte 18 und 19 stufenlos ineinanderübergehen und der Aussendurchmeser der gesamten Spirale gleichbleibend ist.

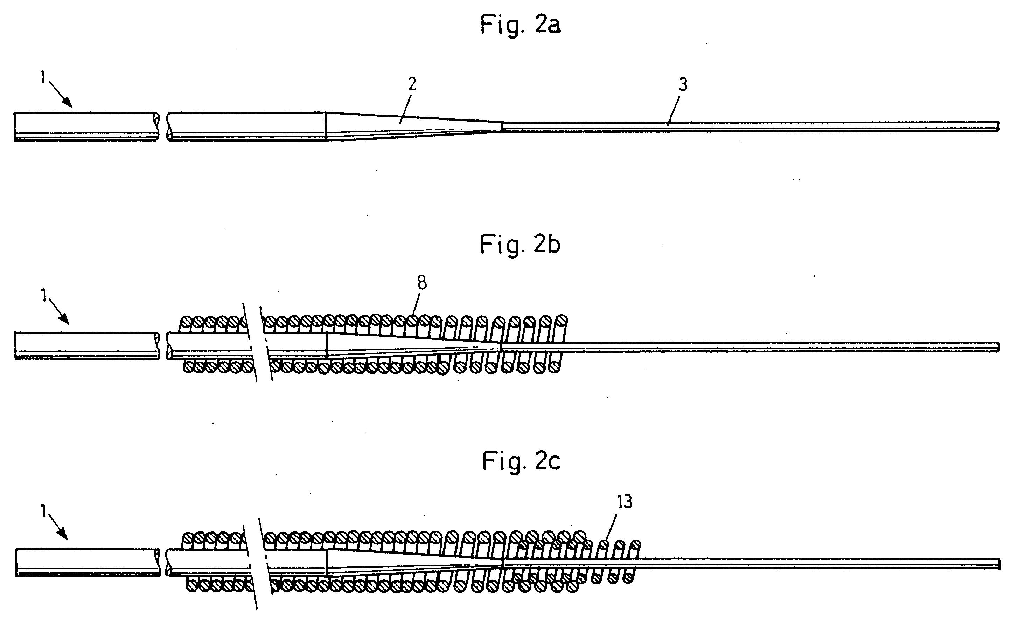

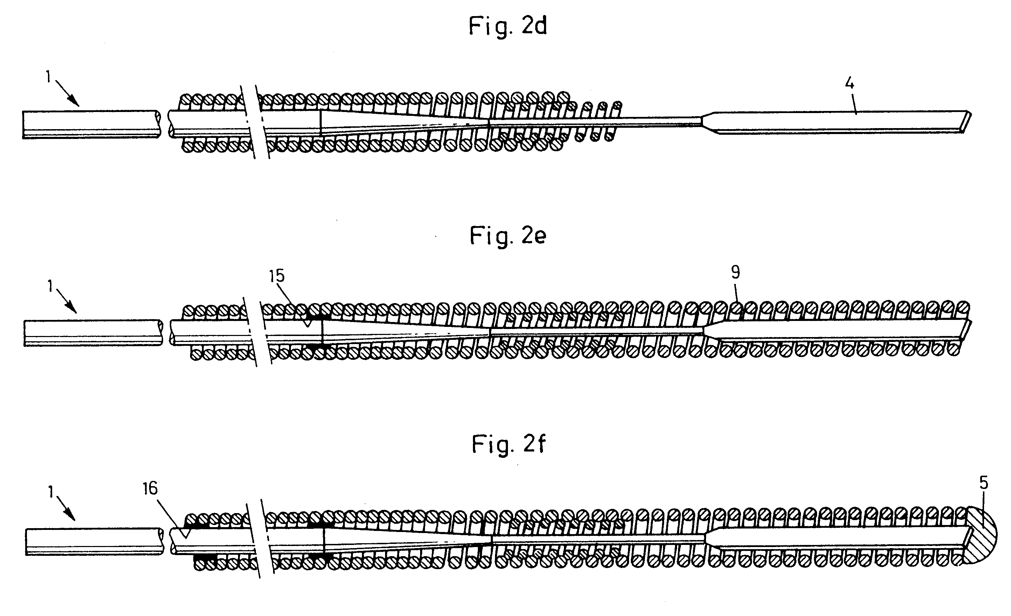

Anhand der Figuren 2a bis 2i wird nachfolgend die Herstellung des erfindungsgemässen Führungsdrahtes kurz erläutert.

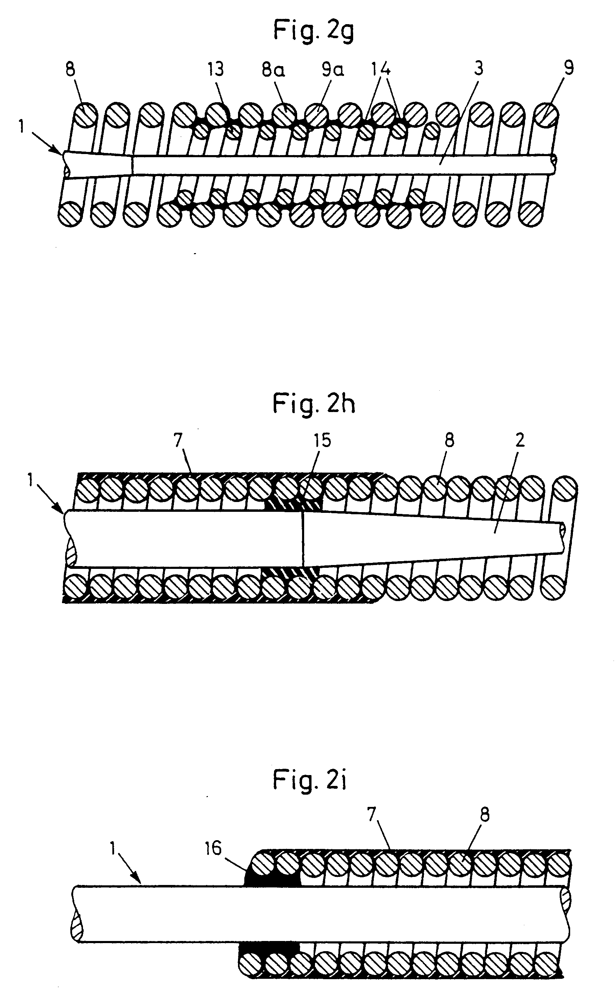

Auf den Schaft 1 wird gemäss den Figuren 2a und 2b vom distalen Ende her der proximale Teil 8 aufgeschoben und in das distale Ende des proximalen Teils 8 die Verbindungsspirale 13 mit beispielsweise vier Windungen eingedreht. Die kreisrunde Spitze des Schaftes 1 wird nun flachgepresst und erhält etwa die in Fig. 2d gezeigte Form. Im nächsten Verfahrensschritt wird der distale Teil 9 auf den Schaft 1 aufgeschoben und auf die Verbindungsspirale 13 aufgedreht. Durch Plasma-Schweissen oder Verlöten der distalen Spitze wird der distale Teil 9 mit dem Schaft 1 fest verbunden, und die dadurch entstandene Spitze 5 vorne abgerundet. Die Verbindungsspirale 13 wird nun mit den Teilen 8 und 9 verlötet. Wie die Fig. 2g zeigt, erstreckt sich die Lötstelle 14 im wesentlichen über die gesamte Länge der Verbindungsspirale 13. Zur besseren Torsionsübertragung wird gemäss Fig. 2h eine Leimstelle 15 angebracht, welche den Schaft 1 mit dem proximalen Teil 8 verbindet. Schliesslich wird der proximale Teil 8 an seinem proximalen Ende mit dem Schaft 1 verlötet, so dass die in Fig. 2i gezeigte Lötstelle 16 entsteht. Diese Lötstelle 16 wird vorzugsweise zuletzt hergestellt, damit die Spirale 6 absolut spannungsfrei über dem Schaft 1 liegt.

## Patentansprüche

1. Führungsdraht, insbesondere zum perkutanen Einführen eines Ballondilatationskatheters in ein Blutgefäss, mit einem länglichen flexiblen Schaft (1), der ein proximales Ende und ein sich verjüngendes distales Ende (2,3,4) aufweist, mit einer flexiblen Spirale (6), welche das distale Ende (2,3,4) des Schaftes (1) umgibt und mit diesem verbunden ist, wobei die Spirale (6) einen distalen röntgensichtbaren Teil (9) und einen proximalen röntgenunsichtbaren Teil (8) aufweist, und mit Verbindungsmitteln (13) zum Verbinden der beiden Teile (8,9) der Spirale (6) miteinander, wobei die Verbindungsmittel (13) an einem Ende in den distalen Teil (9) und an einem anderen Ende in den proximalen Teil (8) der flexiblen Spirale (6) eingedreht sind, dadurch gekennzeichnet, dass die Verbindungsmittel (13) eine Verbindungsspirale umfassen, die an ihren beiden Enden vom proximalen (8) bzw. distalen (9) Teil der flexiblen Spirale (6) überdeckt ist, wobei diese Enden jeweils aussenseitig mit der Innenseite des proximalen bzw. distalen Teils (8,9) der flexiblen Spirale (6) in Gewindeeingriff sind.

2. Führungsdraht nach Anspruch 1, dadurch gekennzeichnet, dass der proximale Teil (8) und der distale Teil (9) im Bereich der Verbindungsspirale (13) in Längsrichtung gedehnt sind, der durch die Dehnung entstehende Spalt jedoch kleiner ist als der Durchmesser des Verbindungsspiraldrahtes.

3. Führungsdraht nach Anspruch 1 oder 2, dadurch gekennzeichnet, dass die Verbindungsspirale (13) in ihrer Längsrichtung gedehnt ist.

4. Führungsdraht nach einem der Ansprüche 1 bis 3, dadurch gekennzeichnet, dass Drähte (18,19) des proximalen Teils und des distalen Teils (9) im Bereich der Verbindungsspirale (13) stumpf aneinanderstossen (Fig. 1b).

5. Führungsdraht nach einem der Ansprüche 1 bis 4, dadurch gekennzeichnet, dass die Verbindungsspirale (13) mehrere Windungen, vorzugsweise etwa sechs bis acht Windungen umfasst.

6. Führungsdraht nach einem der Ansprüche 1 bis 5, dadurch gekennzeichnet, dass die Verbindungsspirale (13) aus einem röntgensichtbaren Draht, beispielsweise aus einem vergoldeten Wolframdraht hergestellt ist.

7. Führungsdraht nach Anspruch 6, dadurch gekennzeichnet, dass der Draht der Verbindungsspirale (13) einen Aussendurchmesser von 0,05 bis 0,10 mm aufweist.

8. Führungsdraht nach einem der Ansprüche 1 bis 7, dadurch gekennzeichnet, dass die Verbindungsspirale (13) mit dem distalen Teil (9) und dem proximalen Teil (8) verlötet ist.

9. Führungsdraht nach Anspruch 8, dadurch gekennzeichnet, dass die Verbindungsspirale (13) entlang ihrer Aussenseite mit dem distalen Teil (8) und dem proximalen Teil (9) verlötet ist.

10. Führungsdraht nach einem der Ansprüche 1 bis 9, dadurch gekennzeichnet, dass der Schaft (1) an seiner distalen Spitze mittels einer Plasma-Schweissstelle (5) mit der Spiralfeder (6) fest verbunden ist.

11. Führungsdraht nach einem der Ansprüche 1 bis 10, dadurch gekennzeichnet, dass der proximale Teil (8) mittels einer Leimstelle (15) mit dem Schaft (1) verbunden ist.

12. Führungsdraht nach einem der Ansprüche 1 bis 11, dadurch gekennzeichnet, dass der proximale Teil (8) aus einem nichtrostenden Stahldraht (18) hergestellt ist.

13. Führungsdraht nach einem der Ansprüche 1 bis 12, dadurch gekennzeichnet, dass der distale Teil (9) und der proximale Teil (8) den gleichen Aussendurchmesser aufweisen.

## Claims

1. A guide wire, more particularly for the percutaneous introduction of a balloon dilation catheter into a blood vessel, with an elongated flexible shaft (1), which comprises a proximal end and a tapered distal end (2, 3, 4), with a flexible coil (6), which surrounds the distal end (2, 3, 4) of the shaft (1) and is attached thereto, the coil (6) comprising a distal, radiologically visible part (9) and a proximal, radiologically invisible part (8), and with connecting means (13) for joining the two parts (8, 9) of the coil (6) to one another, the connecting means (13) being screwed at one end into the distal part (9) and at the other end into the proximal part (8) of the flexible coil (6), characterised in that the connecting means (13) comprise a connecting coil, which is covered at both ends by the proximal (8) or distal (9) parts of the flexible coil (6), the said ends lying in threaded engagement on their outside with the inside of the proximal or distal part (8, 9) of the flexible coil (6).

2. A guide wire according to claim 1, characterised in that the proximal part (8) and the distal part (9) are stretched in the longitudinal direction in the region of the connecting coil (13), although the gap produced by the stretching is smaller than the diameter of the connecting coil wire.

3. A guide wire according to claim 1 or 2, characterised in that the connecting coil (13) is stretched in its longitudinal direction.

4. A guide wire according to one of claims 1 to 3, characterised in that wires (18, 19) of the proximal part and of the distal part (9) abut bluntly against one another in the region of the connecting coil (13) (Fig. 1b).

5. A guide wire according to one of claims 1 to 4, characterised in that the connecting coil (13) comprises a plurality of turns, preferably approximately six to eight turns.

6. A guide wire according to one of claims 1 to 5, characterised in that the connecting coil (13) is manufactured from a radiologically visible wire, for example for a gold-plated tungsten wire.

7. A guide wire according to claim 6, characterised in that the wire of the connecting coil (13) has an external diameter of 0.05 to 0.10 mm.

8. A guide wire according to one of claims 1 to 7, characterised in that the connecting coil (13) is soldered to the distal part (9) and the proximal part (8).

9. A guide wire according to claim 8, characterised in that the connecting coil (13) is soldered along its outer side to the distal part (8) and the proximal part (9).

10. A guide wire according to one of claims 1 to 9, characterised in that the shaft (1) is securely connected at its distal tip to the coil spring (6) by means of a plasma welding site (5).

11. A guide wire according to one of claims 1 to 10, characterised in that the proximal part (8) is connected to the shaft (1) by means of an adhesive point (15).

12. A guide wire according to one of claims 1 to 11, characterised in that the proximal part (8) is manufactured from a non-corrosive steel wire (18).

13. A guide wire according to one of claims 1 to 12, characterised in that the distal part (9) and the proximal part (8) have the same external diameter.

## Revendications

1. Fil de guidage, notamment pour l'introduction percutanée d'un cathéter à ballonnet dilatable dans un vaisseau sanguin, comportant une tige flexible allongée (1), qui possède une extrémité proximale et une extrémité distale rétrécie (2,3,4), un élément hélicoïdal flexible (6), qui entoure l'extrémité distale (2,3,4) de la tige (1) et est relié à cette dernière, l'élément hélicoïdal (6) possédant une partie distale (9) visible en radiographie et une extrémité proximale (8) non visible en radiographie, et des moyens de liaison (13) pour relier les deux parties (8,9) de l'élément hélicoïdal (6) entre elles, les moyens de liaison (13) étant vissés, par une extrémité, dans la partie distale (9) et, par son autre extrémité, dans la partie proximale (8) de l'élément hélicoïdal flexible (6), caractérisé en ce que les moyens de liaison (13) comprennent un élément hélicoïdal de liaison, qui est recouvert, au niveau de ses deux extrémités, par la partie proximale (8) et par la partie distale (9) de l'élément hélicoïdal flexible (6), ces extrémités engrenant par vissage, respectivement au niveau de leur face extérieure, avec le côté intérieur des parties proximale et distale (8,9) de l'élément hélicoïdal flexible (6).

2. Fil de guidage selon la revendication 1, caractérisé en ce que l'extrémité proximale (8) et l'extrémité distale (9) sont étirées dans la direction longitudinale, dans la zone de l'élément hélicoïdal de liaison (13), mais que la fente formée par l'étirage a une taille inférieure au diamètre du fil de l'élément hélicoïdal de liaison.

3. Fil de guidage selon la revendication 1 ou 2, caractérisé en ce que l'élément hélicoïdal de liaison (13) est étiré dans sa direction longitudinale.

4. Fil de guidage selon l'une des revendications 1 à 3, caractérisé en ce que des fils (18,19) de la partie proximale et de la partie distale (9) sont placés en aboutement dans la zone de l'élément hélicoïdal de liaison (13) (figure 1b).

5. Fil de guidage selon l'une des revendications 1 à 4, caractérisé en ce que l'élément hélicoïdal de liaison (13) comporte plusieurs spires, de préférence environ six à huit spires.

6. Fil de guidage selon l'une des revendications 1 à 5, caractérisé en ce que l'élément hélicoidal de liaison (13) est constitué par un fil visible en radiographie, par exemple un fil de tungstène plaqué d'or.

7. Fil de guidage selon la revendication 6, caractérisé en ce que l'élément hélicoïdal de liaison (13) possède un diamètre extérieur compris entre 0,05 et 0,10 mm.

8. Fil de guidage selon l'une des revendications 1 à 7, caractérisé en ce que l'élément hélicoïdal de liaison (13) est fixé par brasage à la partie distale (9) et à la partie proximale (8).

9. Fil de guidage selon la revendication 8, caractérisé en ce que l'élément hélicoïdal de liaison (13) est fixé par brasage, le long de sa face extérieure, à la partie distale (9) et la partie proximale (8).

10. Fil de guidage selon l'une des revendications 1 à 9, caractérisé en ce que la tige (1) est reliée rigidement, au niveau de sa pointe distale, au ressort hélicoïdal (6), au moyen d'une zone de soudage au plasma (5).

11. Fil de guidage selon l'une des revendications 1 à 10, caractérisé en ce que la partie proximale (8) est reliée à la tige (1) au moyen d'un point de colle (15).

12. Fil de guidage selon l'une des revendications 1 à 11, caractérisé en ce que la partie proximale (8) est réalisée en un fil d'acier inoxydable (18).

13. Fil de guidage selon l'une des revendications 1 à 12, caractérisé en ce que la partie distale (9) et la partie proximale (8) possèdent le même diamètre extérieur.
